# EUROPÄISCHE PATENTSCHRIFT

(11) **EP 3 923 799 B1**
(45) Veröffentlichungstag und Bekanntmachung des Hinweises auf die Patenterteilung: **25.09.2024**
(21) Anmeldenummer: 20705189.7
(22) Anmeldetag: 13.02.2020
(51) Int. Cl.: A61B 5/053, A61B 5/00

(54) **MESSGERÄT ZUR ERMITTLUNG DES BLUTZUCKERGEHALTS**
MEASURING DEVICE FOR DETERMINING BLOOD SUGAR CONTENT
APPAREIL DE MESURE PERMETTANT DE DÉTERMINER LA GLYCÉMIE

(30) Priorität: 13.02.2019 DE 102019103641
(43) Veröffentlichungstag der Anmeldung: 22.12.2021
(73) Patentinhaber: Gericke Estermann, Monika, 8032 Zürich (CH)
(72) Erfinder: TORKOVSKI, Ivan Ivanowitsch, 8702 Zollikon (CH); GERICKE ESTERMANN, Monika, 8032 Zürich (CH)
(74) Vertreter: Kleine, Hubertus
(86) Internationale Anmeldenummer: PCT/EP2020/053760
(87) Internationale Veröffentlichungsnummer: WO 2020/165344

(56) Entgegenhaltungen:
- EP-A1- 2 344 025
- US-A1- 2012 209 101
- US-B1- 6 408 200
- US-B1- 7 215 989
- ROMAN KUSCHE ET AL: "Dry electrodes for bioimpedance measurements -- design, characterization and comparison", IOP BIOMEDICAL PHYSICS & ENGINEERING EPRESS, vol. 5, no. 1, 8 November 2018 (2018-11-08), XP081513995, DOI: 10.1088/2057-1976/AAEA59

## Beschreibung

Die vorliegende Erfindung betrifft ein Messgerät zur Ermittlung des Blutzuckergehalts nach dem Oberbegriff des Anspruchs 1.

Aus der EP 2 344 025 A1 ist eine Vorrichtung zur Bestimmung des Blutzuckergehaltes bzw. zur Bewertung einer Glukoseänderung im Blut eines Menschen bekannt.

Aus der EP 2 344 025 A1 ist der Einsatz balkenförmiger Elektroden bekannt. Elektroden für das vorgenannte Anwendungsgebiet bestehen typischerweise aus einem widerstandsfähigen Metall, welches hohe Verschleißfestigkeit und auch hohe chemische Inertheit gegenüber Oxidation aufweist. In Betracht kommen somit beispielsweise Edelstahlelektroden.

Die Sensoreinheit der EP 2 344 025 A1 benötigt allerdings aufgrund der Ausgestaltung der Elektroden vergleichsweise viel Platz. Die Größe der Kontaktfläche sollte dabei nicht zu klein gewählt werden, um eine Messwerterfassung zu gewährleisten, wobei die Leitfähigkeit der Edelstahlelektroden berücksichtigt werden muss.

Verschiedene Elektrodenvarianten von Messgeräten mit Hautkontakt werden sodann in der Veröffentlichung Kutsche et al. "Dry electrodes for bioimpedance measurement.." Biomedical physics & engineering epress, 8.Nov.2018, sowie in der US 7 215 989 B1, der US 6 408 200 B1 und der US 2012/209101 A1 offenbart.

Ausgehend von der vorgenannten Problemstellung ist es nunmehr die Aufgabe der vorliegenden Erfindung eine optimale Erfassung der Impedanzdaten zu erreichen.

Die vorliegende Erfindung löst diese Aufgabe durch das Bereitstellen eines Messgeräts mit den Merkmalen des Anspruchs 1.

Ein erfindungsgemäßes Messgerät dient der Ermittlung eines Schätzwertes für den Blutzuckergehalt einer Person durch eine noninvasive Impedanzmessung. Die Impedanzmessung ermöglicht über eine Aussage zur Volumenänderung der interstitiellen Flüssigkeitskompartimente und somit des osmotischen Drucks. Dieser wiederum ändert sich je nach dem Blutzuckerspiegel. Das Messgerät misst insbesondere den Glucosegehalt im interstitiellen Gewebe.

Zur Auswertung der Impedanzmessung verfügt das Messgerät über einen Messumformer, welcher eine Änderung des Blutzuckergehalt aus einem Messwert der Impedanzmessung ermitteln kann.

Weiter verfügt das Messgerät über einen Messaufnehmer in Form einer Sensoreinheit zum Erfassen zumindest eines Messwertes, insbesondere mehrerer unterschiedlicher Messwerte. Die Messung kann diskontinuierlich in Intervallen oder kontinuierlich erfolgen. Das Messgerät kann vorzugsweise batteriebetrieben sein.

Die Sensoreinheit weist zumindest zwei tetrapolare Elektrodenanordnungen auf mit jeweils zumindest zwei Elektrodenpaaren, also vier Elektroden, welche entlang einer ersten linearen Achse angeordnet sind.

Die Achsen der zwei tetrapolaren Elektrodenanordnungen sind senkrecht zueinander ausgerichtet. Ein erstes Elektrodenpaar dient zum Aussenden und Empfang eines Stromsignals und ein zweites Elektrodenpaar dient dem Abgriff eines Potentials bei Kontakt mit der Haut der Person.

Zumindest eine der Elektroden der Sensoreinheit, insbesondere alle Elektroden der tetrapolaren Elektrodenanordnungen, weisen eine Kontaktfläche zur Kontaktierung mit der Haut auf, welche aus einem Metall oder einer Metalllegierung mit einer Leitfähigkeit von mehr als 1*10⁷ S/m besteht. Die Leitfähigkeit bezieht sich auf eine Messung unter Standardbedingungen, also bei einer Temperatur von 20°C und Normaldruck.

Durch die Materialauswahl des Elektrodenmaterials mit der vorgenannten hohen Leitfähigkeit kann ein zuverlässiger Signalempfang gewährleistet werden und zugleich kann die Kontaktfläche der Elektroden auf ein Minimum reduziert werden. Dadurch lassen sich im Bereich der Sensoreinheit viele Elektroden anordnen, ohne dass es zu einem Kurzschluss zwischen den Elektroden kommt.

Die Elektrode weist erfindungsgemäß eine Innenelektrode auf, welche aus einem Edelmetall, insbesondere aus Gold, besteht, und eine Stützhülse aus einem Material mit einer Vickershärte von mehr als 40, gemäß der DIN EN 6507-1 :2018-07. Die Stützhülse schützt die Innenelektrode mechanischem Verschleiß.

Die Stützhülse kann auch aus einer Siliziumverbindung mit einer Wärmeleitfähigkeit von mehr als 100 W/(m*K) bestehen. Besonders bevorzugt entspricht das Material der Stützhülse auch in diesem Fall einer Vickershärte von mehr als 40. Alle Wärmeleitfähigkeiten in der vorliegenden Erfindung beziehen sich auf eine Messung bei 0°C und einer Luftfeuchte von 50%.

Bevorzugte Ausgestaltungen der Erfindung sind Gegenstand der Unteransprüche.

Bevorzugt kann das Metall der Kontaktfläche aus Gold, Rhodium, Tantal, Iridium, Wolfram und/oder Osmium ausgebildet sein. Alternativ kann die Metalllegierung zumindest zu 40 Gew.% aus einem oder mehreren der vorgenannten Metalle gebildet sein.

Die Elektrode kann einen Elektrodenkörper aus Kupfer und/oder Messing und/oder einer Siliziumverbindung, insbesondere alpha-Siliziumcarbid, aufweisen, wobei der Korpus mit einer Beschichtung aus Rhodium, Tantal, Iridium und/oder Osmium unter Ausbildung der Kontaktfläche versehen ist. Dadurch weist die Elektrodenfläche eine chemisch inerte Kontaktfläche auf. Bei dieser Variante der Elektrode kann die Stützhülse entfallen.

Besonders bevorzugt ist eine Kombination aus Kupfer oder Bronze als Innenelektrode und Rhodium als Beschichtung.

Die vorgenannte Beschichtung kann eine Beschichtungsdicke von zumindest 2 µm, vorzugsweise zwischen 3 bis 10 µm, über die gesamte Kontaktfläche aufweisen.

Die Sensoreinheit kann eine Platte oder ein Ledersegment aufweisen, auf welcher oder welchem die tetrapolaren Elektrodenanordnungen festgelegt, insbesondere verankert, sind, wobei die Platte oder das Ledersegment zur Integrierung in einem flexiblen Armband eine Dauerbiegefestigkeit nach DIN EN ISO 5402-1:2017-05 oder ISO 4666-2 von 100.000 Knickungen ohne Beschädigung aufweist. Dies kann durch Messung mit einem Flexometer nachgewiesen werden.

Jeweils eine Elektrode kann in einer Öffnung der Platte und/oder des Lederstreifens angeordnet sein, wobei die Stützhülse eine radiale Auskragung als Anschlag auf der Oberfläche der Platte oder des Lederstreifens randseitig zu der Öffnung aufweist. Dadurch gelingt eine sichere Positionierung der Elektrode an oder in der Platte oder im Lederstreifen. Beispielsweise kann auch ein Kunststoff, z.B. ein Gummi, oder Alcantara als Material der Platte genutzt werden.

Die maximale Erstreckung der Kontaktfläche wird nachfolgend als Breite bezeichnet. Diese Breite, im Fall einer Rundelektrode entspricht dies dem Durchmesser, der Kontaktfläche der Elektrode, insbesondere der Innenelektrode, kann geringer sein als 5 mm, vorzugsweise 2-3 mm.

Die Elektrode oder zumindest die Innenelektrode kann insbesondere als Rundelektrode, insbesondere als Pilzkopfelektrode, ausgebildet ist. Rundelektrode mit einer Kontaktfläche welche symmetrisch in alle Richtungen ist, ermöglicht eine exaktere Ermittlung von Impedanz-Anisotropien.

Die Sensoreinheit kann vorzugsweise vier oder acht tetrapolare Elektrodenanordnungen mit jeweils zwei Elektrodenpaaren, welche auf einer linearen Achse angeordnet sind, aufweisen.

Die Elektroden der Sensoreinheit können auf zwei, vorzugsweise konzentrisch-zueinander angeordneten, Kreisbahnen angeordnet sein, wobei die Zahl der Elektroden auf der ersten Kreisbahn gleich der Zahl der Elektroden auf der zweiten Kreisbahn ist.

Der Durchmesser der ersten Kreisbahn kann zwischen 20 bis 40 mm betragen und der Durchmesser der zweiten Kreisbahn kann zumindest um 30% kleiner sein als der Durchmesser der ersten Kreisbahn.

Der Abstand zweier benachbarter Elektroden zweier benachbarter Elektrodenanordnungen kann mehr als 4 mm, vorzugsweise zwischen 5 und 8 mm, betragen, um eine Signalübertragung zu vermeiden.

Die Anordnung der Elektroden der Sensoreinheit kann punktsymmetrisch und insbesondere spiegelsymmetrisch sein.

Bevorzugt ist Messgerät als ein portables Messgerät ausgebildet mit einem Armband und/oder Fußband t, wobei die Sensoreinheit eine Breite parallel zur Armband- und/oder Fußbandbreite von weniger als 40 mm, vorzugsweise zwischen 15-35 mm aufweist.

Die Sensoreinheit kann ein starr mit der Person verbindbares, vorzugsweise plattenförmiges, Element aufweisen, wobei die Elektroden der Elektrodenanordnungen, insbesondere die Achsen der Elektrodenanordnungen, drehbar um eine Drehachse gegenüber dem plattenförmigen Element angeordnet sind.

Das plattenförmige Element kann z.B. eine Leiterplatte sein. Zur Fixierung der Elektroden bei gleichbleibendem Elektrodenabstand auf einer Kreisbahn empfiehlt sich eine Grundplatte oder eine Ringplatte oder eine Kombination aus beiden

Das vorgenannte Element kann vorteilhaft kreisbogenförmige Kontaktflächen zum Signalabgriff der Elektroden aufweisen.

Zum Zweck einer virtuellen Drehung kann die Achse einer der tetrapolaren Elektrodenanordnungen mit einer zweiten Achse einer weiteren Elektrodenanordnung, ausgehend von einer gemeinsamen Elektrode als Winkelscheitel winklig zueinander angeordnet sein, wobei der Signalabgriff durch eine Steuereinheit derart gesteuert wird, dass zum Zweck der virtuellen Drehung der Elektrodenanordnung wahlweise die Signale an den Elektroden der ersten oder der zweiten Achse abgegriffen werden.

Weitere Vorteile, Merkmale und Einzelheiten der Erfindung ergeben sich aus der nachfolgenden Beschreibung, in der mehrere Ausführungsbeispiele der Erfindung anhand der beiliegenden Zeichnungen näher erläutert werden. Der Fachmann wird die in den Zeichnungen, der Beschreibung und den Ansprüchen in Kombination offenbarten Merkmale zweckmäßigerweise auch einzeln betrachten und zu sinnvollen weiteren Kombinationen zusammenfassen. Es zeigen:
- Fig. 1: schematische Darstellung eines Glucosemessgerätes;
- Fig. 2: Darstellung einer ersten Variante einer Elektrodenanordnung zum Hautkontakt; und
- Fig. 3: Schnittdarstellung einer Elektrode; und
- Fig. 4: Draufsicht auf eine Elektrode;
- Fig. 5a)-c): Draufsicht auf eine zweite Variante einer Sensoreinheit zum Hautkontakt;
- Fig. 6a)-c): Draufsicht auf eine dritte Variante einer Sensoreinheit zum Hautkontakt;
- Fig. 7a)-c): Draufsicht auf eine vierte Variante einer Sensoreinheit zum Hautkontakt;
- Fig. 8: Draufsicht auf eine fünfte Variante einer Sensoreinheit zum Hautkontakt;
- Fig.9: Draufsicht auf eine sechste Variante einer Sensoreinheit zum Hautkontakt;
- Fig. 10a)-b): Draufsicht auf Mess- und Kompensationsachsen für die Sensoreinheiten der Fig. 8 und 9.

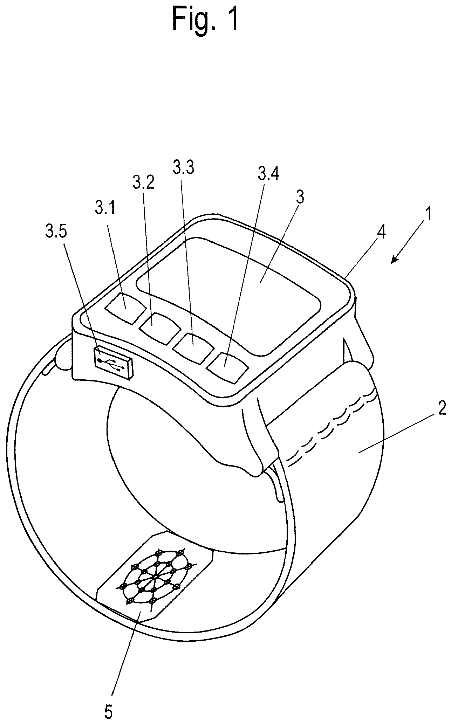

Fig. 1 zeigt eine schematische Darstellung eines Messgeräts 1 zur Ermittlung einer Änderung des Blutzuckergehalts. Dieses Messgerät kann bei bekanntem Angangsgehalts sogar den Blutzuckergehalt als Absolutwert ermitteln. Eine typische Ausgestaltung des erfindungsgemäßen Messgerätes ist ein Glucosemessgerät 1, dessen Wirkweise u. a. bereits in der EP 2 344 025 A1 beschrieben wird. Dieses Gerät dient zur Ausführung eines nichtinvasiven Verfahrens zur Schätzung des Glucosespiegels im Blut einer Person.

Dieser Glucosespiegel ist durch eine Impedanzmessung ermittelbar, wobei zunächst eine Messung der Impedanz der Haut erfolgt. Hierfür geht man von einer Anordnung von zumindest zwei Elektroden, einer Sende- und einer Empfangselektrode, aus. Dabei wird ein elektrischer Strom zwischen den Elektroden über die Haut übertragen und zugleich eine Potentialdifferenz ermittelt, welche sich aufgrund der Haut im Zwischenraum der Elektroden ergibt. Die ermittelten Ströme und Potentiale können zur Ermittlung eines Schätzwertes für einen Glucosewert, analog zur EP 2 344 025 A1, genutzt werden.

Das Glucosemessgerät 1 umfasst ein Armband 2 zur Festlegung des Glucosemessgerätes an einem Arm, insbesondere einem Handgelenk. Alternativ ist auch die Anbringung an einem Fußgelenk bzw. Fußknöchel oder an einer anderen Körperstelle möglich. In diesen Bereichen liegt auch eine sehr große Anisotropie der körpereignen Impedanz vor.

Das Armband kann analog zum Armband einer Armbanduhr aus Metall, Leder oder einem textilen Material bestehen, wobei in das Armband bevorzugt ein Signalleiter, z. B. eine Flexprint-Leiterplatte, eingearbeitet ist.

Das Glucosemessgerät 1 umfasst einen Messumformer 4 mit einer Auswerteelektronik zur Auswertung von Impedanz-Messdaten und zur Ermittlung von Informationen hinsichtlich eines aus den Messdaten ermittelten Glucosespiegels. Weiter umfasst der Messumformer 4 ein Display 3 zur Ausgabe der vorgenannten Informationen.

Unterhalb des Displays 3 sind mehrere Bedienelemente 3.1-3.4, z. B. Tasten, angeordnet, mit welchem mehrere Anzeigeoptionen auswählbar sind. Weiterhin sind Sollwerte oder andere Menüelemente über die Bedienelemente einstellbar.

Ein weiteres randseitiges Bedienelement 3.5 ermöglicht die Aktivierung einer Funkverbindung, vorzugsweise über Bluetooth, zur Datenübertragung z. B. an einen Computer. So können beispielsweise Analysewerte einer vorab entnommenen interstitiellen Flüssigkeitskompartimente, z.B. die Leitfähigkeit und/oder die Zusammensetzung, zur Kalibration des Glucosemessgerätes zur Verbesserung der Genauigkeit des ermittelten Schätzwertes übertragen werden. Das Glucosemessgerät benötigt allerdings zur Ermittlung eines Schätzwertes nicht zwingend diese Werte, sondern es geht von einer Grundeinstellung mit voreingestellten Sollwerten aus.

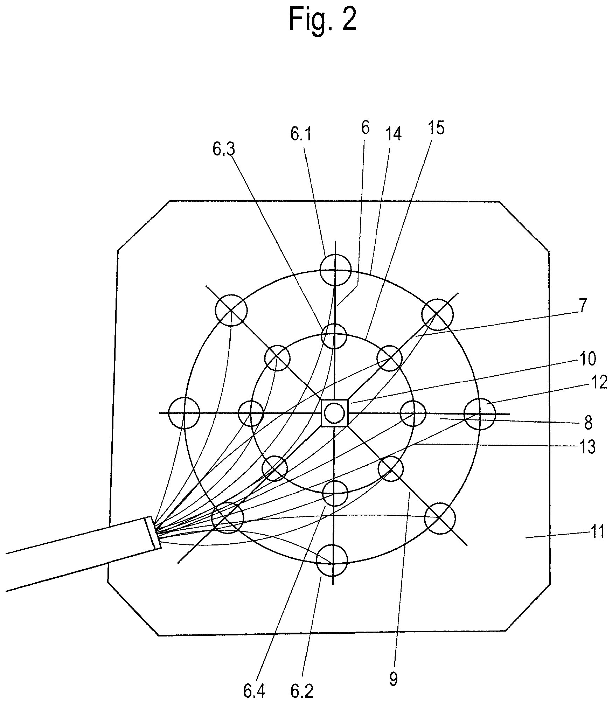

Fig. 2 zeigt eine Sensoreinheit 5, welche an dem Armband 2 der Fig. 1 anordenbar ist, wobei die Sensoreinheit 5 eine Anordnung aus mehreren Elektroden 6 umfasst.

Die Sensoreinheit 5 umfasst vier sogenannte tetrapolare Elektrodenanordnungen 6, 7, 8 und 9 mit jeweils zwei Elektrodenpaaren, welche in einer spiegelsymmetrischen Anordnung zueinander angeordnet sind. Der zu erfassende Hautbereich ist dabei zwischen den Elektroden der Elektrodenanordnungen 6-9 der Sensoreinheit 5 eingeschlossen. Jeweils eine Elektrodenanordnung 6.1-6.4 liegt dabei auf einer Spiegelsymmetrieachse A und weist zwei Elektrodenpaare 6.1 und 6.2 oder 6.3 und 6.4 auf, zwischen welchen es zu einer Interferenzbildung kommt.

Das erste Elektrodenpaar umfasst Elektroden 6.1 und 6.2 zum Aussenden und Empfang eines Stromsignals.

Über ein zweites Elektrodenpaar, umfassend die Elektroden 6.3 und 6.4, erfolgt ein Abgriff eines Potentials bei Kontakt mit der Haut.

Die jeweiligen Spiegelsymmetrieachsen A-D der tetrapolaren Elektrodenanordnungen 6-9 sind punktsymmetrisch um einen Mittelpunkt 10 der Sensoreinheit 5 mit einem Versatz von 45° angeordnet. Optional kann im Bereich des Mittelpunkts ein Temperatursensor angeordnet sein.

Die Elektroden der Elektrodenanordnungen 6-9 sind auf einer dielektrischen Platte 11 angeordnet, welche eine geringere Leitfähigkeit als die Haut aufweist, so dass das Stromsignal durch die Haut übertragen wird und nicht durch einen Kurzschluss zwischen den Elektroden.

Entscheidend für die Messgenauigkeit der Sensoreinheit 5 sind dabei u. a. die Anzahl der Elektrodenanordnungen, die Dimension einer Kontaktfläche 12 der jeweiligen Elektrode mit der Haut und deren Abstand zu einer benachbarten Elektrode.

Die Elektroden der Elektrodenanordnung 6-9 weisen auf ihrer Kontaktfläche 12 entgegengesetzten Seite jeweils eine elektrische Leitung 13 zur Stromversorgung der Elektroden und zum Abgriff der empfangenen Messsignale auf.

Der Abstand einer Elektrode 6.1 oder 6.2 des ersten Elektrodenpaars einer ersten Elektrodenanordnung 6 zu einer Elektrode eines ersten Elektrodenpaares einer benachbarten zweiten Elektrodenanordnung 7 beträgt dabei ausgehend vom Mittelpunkt einer Kontaktfläche 12 einer Elektrode zum Mittelpunkt der Kontaktfläche 12' der benachbarten Elektrode mehr als 8 mm, vorzugsweise zwischen 10 und 15 mm. Selbstverständlich sind die vorgenannten Größen nur bevorzugt und die Abstände können auch größer oder kleiner sein.

Der Abstand einer Elektrode 6.3 oder 6.4 des zweiten Elektrodenpaars einer ersten Elektrodenanordnung 6 zu einer Elektrode eines zweiten Elektrodenpaares einer benachbarten zweiten Elektrodenanordnung 7 beträgt dabei ausgehend vom Mittelpunkt einer Kontaktfläche 12 einer Elektrode zum Mittelpunkt der Kontaktfläche 12' der benachbarten Elektrode mehr als 4 mm, vorzugsweise zwischen 5 und 8 mm. Selbstverständlich sind die vorgenannten Größen nur bevorzugt und die Abstände können auch größer oder kleiner sein.

Die Elektroden des ersten Elektrodenpaares 6.1 und 6.2 sind dabei einander diametral gegenüberliegend auf einer ersten Kreisbahn 13 angeordnet und die Elektroden 6 des zweiten Elektrodenpaares 6.3 und 6.4 sind dabei einander diametral gegenüberliegend auf einer zweiten Kreisbahn 14 angeordnet.

Die beiden Kreisbahnen sind konzentrisch zueinander ausgerichtet, wobei der Durchmesser der ersten Kreisbahn zwischen 20 bis 40 mm beträgt und wobei der Durchmesser der zweiten Kreisbahn 14 zumindest um 30% kleiner ist als der Durchmesser der ersten Kreisbahn 13. Selbstverständlich sind die vorgenannten Durchmesser nur bevorzugt und können auch größer oder kleiner sein.

Auf jeder Kreisbahn 13 und 14 sind acht Elektroden in äquidistanten Abständen zueinander angeordnet.

Die minimale Anzahl an Elektroden pro Kreisbahn ist 8. In diesem Fall sind nur zwei tetrapolare Elektrodenanordnungen mit jeweils vier Elektroden auf einer Symmetrieachse, wobei die Symmetrieachsen senkrecht zueinander angeordnet sind

Die Messgenauigkeit wird allerdings mit der Anzahl der Elektrodenanordnungen erhöht. Aus Platzgründen ist eine Elektrodenzahl von mehr als 32 Elektroden jedoch nicht empfehlenswert.

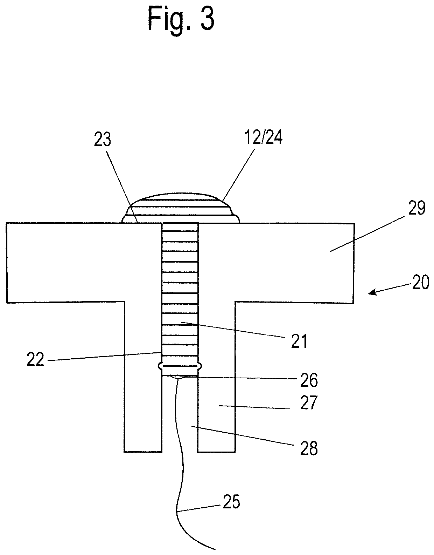

Nachfolgend wird anhand von Fig. 3 der Aufbau einer einzelnen Elektrode 20 der Elektrodenanordnungen 6-9 näher erläutert. Die dargestellte Elektrode 20 kann beispielsweise eine der Elektroden 6.1-6.4 sein. Bevorzugt können alle in Fig. 2 dargestellte Elektroden identisch ausgebildet sein. Diese weisen eine Innenelektrode 21 aus einem Material mit hoher Leitfähigkeit auf.

Wünschenswert für eine Elektrode 20 ist eine möglichst geringe Kontaktfläche, um möglichst viele Elektrodenanordnungen 6-8 punktsymmetrisch nebeneinander auf der Platte 11 anzuordnen. Die Elektrodenfläche hängt u. a. von der elektrischen Stromdichte ab, welche aus physiologischen Gründen auf 1 A/m² begrenzt ist. Bei der in Fig. 3 dargestellten und für den Einsatzzweck größenoptimierte Elektrode 20 kann eine Innenelektrode 21 aus Silber, Rhodium, Iridium, Osmium, Tantal, Kupfer, Gold und/oder Aluminium oder entsprechende metallische Legierungen, wie z. B. Messing, mit einem Anteil an einem oder mehreren der vorgenannten Metalle von zumindest 40 Gew.% aufweisen.

Optimalerweise sollte die elektrische Leitfähigkeit des Materials der Innenelektrode 21 der Elektrode 20 zumindest 1*10⁷ S/m betragen.

Besonders bevorzugt als Material der Innenelektrode 21 oder zumindest für die Kontaktfläche 12 ist dabei Gold, insbesondere Gold mit zumindest 10 Karat, vorzugsweise zumindest 20 Karat und besonders bevorzugt mit zumindest 24 Karat. Dieses Metall ist insbesondere im Unterschied zu Kupfer und Silber, welche eine Tendenz zur Oxidation aufweisen, chemisch inert und somit für den Hautkontakt geeignet.

Auch Rhodium, Iridium, Osmium oder Tantal können als massive Elektroden und/oder als Beschichtungen genutzt werden. Diese Metalle können als Beschichtung auf eine Kupfer- oder Messingelektrode aufgebracht werden, unter Bereitstellung der chemisch-inerten Kontaktfläche 12, unter Ausbildung einer inerten Kontaktfläche. Somit bietet sich eine Innenelektrode 21 mit einem Kupferkorpus und einer endständigen Gold-, Rhodium-, Iridium-, Tantal- und/oder Osmiumbeschichtung unter Ausbildung der Kontaktfläche 12 an.

Die Innenelektrode 21 weist die Form einer sogenannten Pilzkopfelektrode auf, also einen Elektrodenschaft 22 und einen endständig in radialer Richtung aus dem Elektrodenschaft 22 hervorstehenden kappenförmigen Vorsprung 23, welcher eine gebogene Kontaktfläche 24 ausbildet. Der Vorsprung 23 weist einen bevorzugten Durchmesser von weniger als 5 mm, vorzugsweise 2-3 mm, in radialer Richtung, senkrecht zur Längsachse A der Elektrode auf.

An einem entgegengesetzten Ende der Innenelektrode 21 ist eine Signalleitung 25 angeordnet, zur Stromversorgung der Innenelektrode 21 und/oder für einen Signalabgriff von der Innenelektrode 21.

Die Signalleitung 25 kann über eine Lötverbindung 26 mit der Innenelektrode 21 verbunden sein.

Da Gold aber auch die anderen vorgenannten Materialien nur eine geringe Verschleißfestigkeit aufweisen, ist die Innenelektrode 21 durch eine Stützhülse 27 aus einem gegenüber dem Material der Innenelektrode 21 wesentlich verschleißfesteren Material eingefasst. Die Stützhülse kann vorzugsweise aus Titan oder Stahl, vorzugsweise aus Edelstahl, oder einer Legierung der vorgenannten Metalle gebildet sein. Alternativ kann die Stützhülse 27 auch aus einer Keramik oder aus einem Kunststoffmaterial gebildet sein. Insbesondere Siliziumcarbid bietet sich aufgrund seiner optimalen Wärmeleitfähigkeit und seiner großen Härte als Material der Stützhülse an. Auch eine berylliumhaltige Keramik kommt als Material für die Stützhülse in Betracht. Das Siliziumcarbid als auch die berylliumhaltige Keramik kann auch im Verbund mit anderen Materialien, z.B. Metallen, eingesetzt werden. So ist es denkbar, dass die Stützhülsen innen die zylindrische silizium- oder serylliumhaltige Abschnitte und radial nach außen einen zylindrischen metallischen Abschnitt, z.B. aus Edelstahl, aufweist. Dieser zylindrische metallische Abschnitt kann das nichtmetallische Material im Inneren, z.B. bei Rissen im Keramikmaterial, nach außen hin stabilisieren. Die Stützhülse 27 weist einen inneren Kanal 28 parallel zur Längsachse A der Elektrode 20 auf, wobei die Innenelektrode 21 diesen Kanal 28 zumindest einseitig verschließt.

Sie weist eine radiale Auskragung 28 im Bereich der Kontaktfläche 12 der Innenelektrode auf, welche auf der Platte 11 aufliegt und welche als Stützfläche für den kappenförmigen Vorsprung 23 dient. In Fig. 3 zeigt die Stützhülse zudem eine Nut oder einen bereichsweise oder umlaufend-ausgebildeten Hinterschnitt auf, welcher der Verankerung der Innenelektrode in Längsrichtung dient.

Die Platte 11 in welcher die Elektroden angeordnet sind kann vorzugsweise als eine chemisch-inerte und elektrisch-isolierende Kunststoffplatte oder eine Lederplatte ausgebildet sein. Diese sollte eine gewisse Flexibilität aufweisen. Als Materialien bieten sich z. B. Leder und/oder Silikon an. Die Materialwahl der Platte 11 kann von den Klimabedingungen im jeweiligen Einsatzgebiet wie z. B. Luftfeuchte, Temperatur, Auftreten von Sandstürmen und dergleichen abhängen.

Die Messung sollte nicht nur die Oberflächenschichten der Haut, sondern auch Impedanz-Anisotropien in darunter gelegenem Muskelgewebe erfassen. Daher ist es von Vorteil, wenn die Innenelektrode 21 endständig um zumindest 0,5 mm, vorzugsweise zumindest 0,8 mm gegenüber der Oberfläche der Stützhülse 27 hervorsteht.

Im Fall einer beschichteten Kupfer- und/oder Messingelektrode kann durch Aufbringen einer leitfähigen Beschichtung aus Osmium, Rhodium, Iridium die Stützhülse vorteilhaft entfallen.

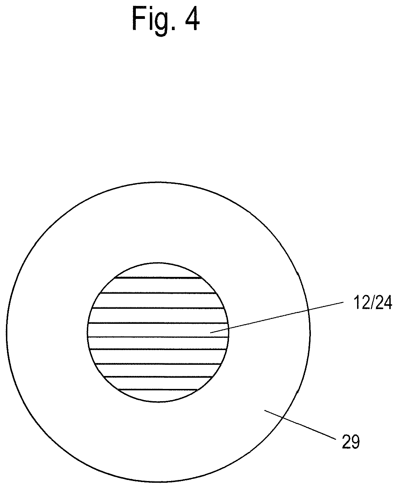

Fig. 4 zeigt eine Draufsicht auf die Elektrode 20 der Fig. 3. Dabei erkennt man, dass der Durchmesser der Auskragung 28 der Stützhülse 27 zumindest um 50 %, vorzugsweise zumindest um 80 %, größer ist als der Durchmesser der Kontaktfläche 12 der Innenelektrode 21.

Die in Fig. 1-4 dargestellten Elektroden sind als Rundelektroden mit runder Kontaktfläche ausgebildet. Es ist allerdings auch möglich die Elektroden streifenförmig, wie in der EP 2 344 025 A1 dargestellt, auszubilden. Es hat sich allerdings gezeigt, dass Rundelektroden besser zur Ermittlung von Impedanz-Anisotropien bei Hautkontakt geeignet sind.

Zur Identifikation bzw. Ermittlung der lokalen Impedanz-Anisotropien in Weichgewebe unterhalb der Haut durch Kontakt mit dem Sensor, ist es von Vorteil, wenn die Kontaktfläche einer einzelnen Elektrode vergleichsweise klein ist. Dies erlaubt eine höhere Anzahl an Sensorachsen. Die kleine Kontaktfläche der einzelnen Elektrode entspricht einer hohen Dichte an elektrischem Strom.

Die hauptsächlichen biologischen Effekte eines hochfrequenten alternierenden Stroms ist ein in-situ Erwärmen des Gewebes unter der Elektrode. Daher ist eine dielektrische Einfassung der Elektrode mit einem Material mit einer hohen Wärmeleitfähigkeit besonders von Vorteil.

Die Mehrzahl an dielektrischen Materialien sind Wärmeisolatoren. Allerdings gibt es einige Ausnahme, welche für die Anwendung geeignet sind. Eine davon ist das künstliche dielektrische Material alpha-Siliziumcarbid mit einer Wärmeleitfähigkeit von mehr als 300 W/(m*K). Zudem ist Siliziumcarbid auch ein sehr hartes und chemisch-inertes Material und daher ideal für die vorbeschriebene Anwendung.

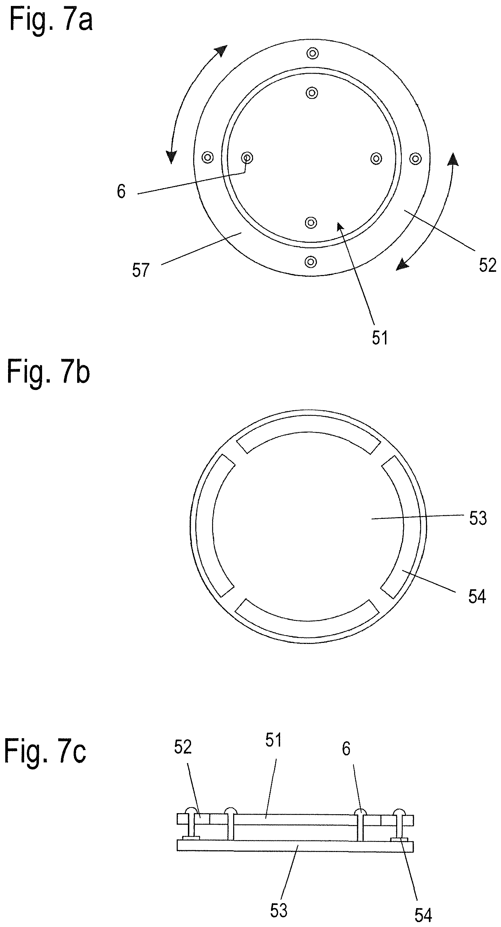

Fig. 7a-c zeigt Einzelteile einer Sensoreinheit, welche rotierbar gelagert ist. Bekannterweise haben nicht alle Menschen eine einheitliche Physiognomie. Dies trifft auch auf die jeweiligen Handgelenke zu. Die Sensoreinheit der Fig. 2 kann als starre Anordnung gegenüber dem Untergrund, z.B. dem Armband, ausgebildet sein, welche auf einen Großteil von Nutzern abgestimmt ist. Bereits die Größe des verwendeten Armbands kann diesbezüglich schon Vorgaben bezüglich des Handgelenks geben. Um einen optimalen Signalabgriff zu erreichen, welcher ggf. auch anatomische Besonderheiten im Bereich des Handgelenks berücksichtigt, kann es im Einzelfall jedoch von Vorteil sein, wenn die Elektrodenanordnung beweglich ist, wobei der Elektrodenabstand der Elektroden zueinander nach Möglichkeit jedoch beibehalten werden sollte.

Daher kann zur patientenindividuellen Anpassung und zur Verbesserung des Signalabgriffs die Elektrodenanordnung rotierbar gegenüber einer Fixierung wie z.B. eines Lederbandes und ggf. auch gegenüber einer Elektronikplatine Signalabgriff gelagert sein.

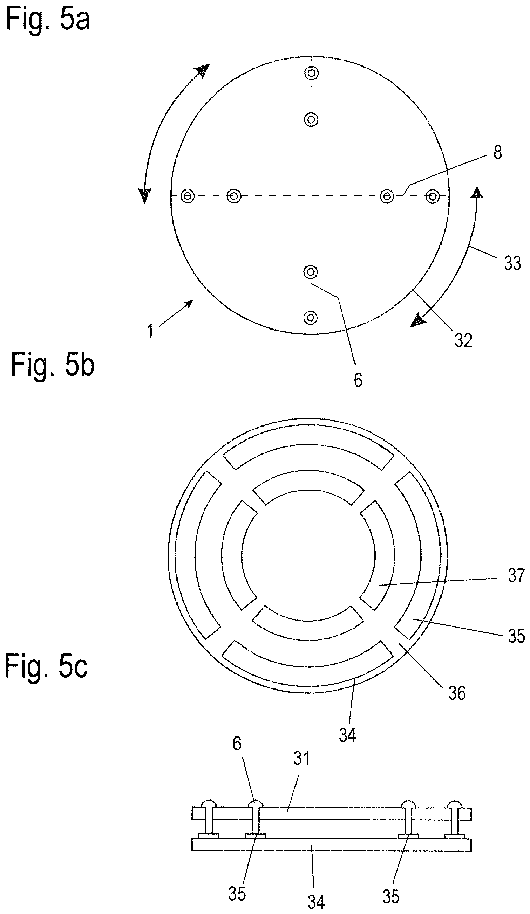

Fig. 5a zeigt die Kontaktseite einer Sensoreinheit mit der Haut in der Draufsicht mit zwei tetrapolare Elektrodenanordnungen 6 und 8. Die Sensoreinheit weist eine Grundplatte 31 und die darin festgelegte Elektroden der besagen Elektrodenanordnungen 6 und 8 auf. Das Material der Elektroden und deren bevorzugte Form wurde bereits zuvor beschrieben.

Grundplatte 31 mit den darin verankerten Elektrodenanordnungen 6 und 8 sind drehbar um den Mittelpunkt der Grundplatte 31 auf einer darunterliegenden Elektronikplatine 34 angeordnet, welche in Fig. 5b dargestellt ist. Die Drehrichtung ist mit dem Pfeil 33 angezeigt. Die Ränder 32 der Grundplatte 31 und der Elektronikplatine 34 sind dabei in dieser Ausführungsvariante bündig zueinander angeordnet. Die Elektronikplatine 34 weist dabei ringsegmentartig-gebogene Kontaktflächen 35 auf der Oberfläche zur Grundplatte 31 hin auf. Diese dienen der elektrischen Kontaktierung mit den Elektroden der Elektrodenanordnungen 6 und 8 und somit dem Signalabgriff. Die Bogenform der Kontaktflächen 35 erlaubt eine Teilrotation der Grundplatte 31 gegenüber der Elektronikplatine 34, ohne dass es dadurch zu einem Kontaktabriss kommt. Zwischen den Kontaktflächen 35 sind Isolierstege vorgesehen, so dass die Kontaktflächen gegeneinander isoliert sind. Die bogenförmigen Kontaktflächen 35 sind auf zwei konzentrisch zueinander angeordneten Kreisbahnen 36 und 37 angeordnet.

Die aus der Schnittansicht in Fig. 5c erkennbar stehen die Kontaktflächen zur gleitenden Kontaktierung der Elektroden aus der Oberfläche der Elektronikplatte 34 hervor. Die Materialdicke der Kontaktflächen 35 von zumindest 10% der Plattendicke der Elektronikplatine 34 ermöglicht einen mechanischen Widerstand der Kontaktflächen.

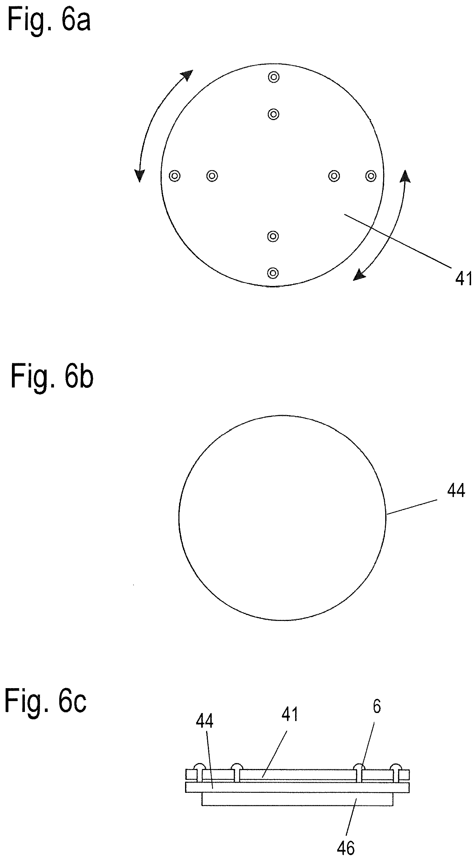

Alternativ zu der in Fig. 5a-c gezeigte Bauweise kann die Bauhöhe der Sensoranordnung auch reduziert werden. Dies ist in Fig. 6a-6c dargestellt. In Fig. 6a ist die Grundplatte 41 und die Elektroden identisch zu Fig. 5a ausgebildet. Im Unterschied zur der Elektronikplatine der Fig. 5b ist in der Fig. 6b eine gedruckte Leiterplatte 44 vorgesehen. Die Kontaktflächen können ebenfalls bogenförmig analog zu Fig. 5b ausgebildet sein. Allerdings sind die Kontaktflächen in dieser Variante in die gedruckte Leiterplatte eingelassen, wodurch sie die Bauhöhe der Gesamtanordnung verringert. Eine zusätzliche Trägerplatte 46 unterstützt die gedruckte Leiterplatte 44 und sorgt für zusätzliche Stabilität.

Die Fig. 7a-7c zeigt eine Variante in welcher anstelle einer einheitlichen Grundplatte eine starr zum Untergrund angeordnete Grundplatte und konzentrisch um die Grundplatte 51 eine Ringplatte 52 angeordnet ist. Die Grundplatte 51 definiert eine erste innere Kreisbahn 56 auf welcher die inneren Elektroden einer jeweiligen Elektrodenanordnung 6 und 8 angeordnet sind, während die Ringplatte 52 eine äußerte Kreisbahn 57 mit den äußeren Elektroden einer jeweiligen Elektrodenanordnung 6 und 8 aufweist. Die Ringplatte 52 ist dabei drehbar gegenüber der Grundplatte 51 angeordnet. Entsprechend weist eine parallel zur Grundplatte 51 angeordnete Leiterplatte 53 bogen- bzw. ringsegmentförmige Kontaktflächen 54 zur Kontaktierung mit den äußeren Elektroden auf der Ringplatte 52 auf. Eine zweite Kreisbahn mit bogenförmigen Kontaktflächen für die Elektroden der Grundplatte 51 ist hingegen nicht vorgesehen und auch nicht notwendig.

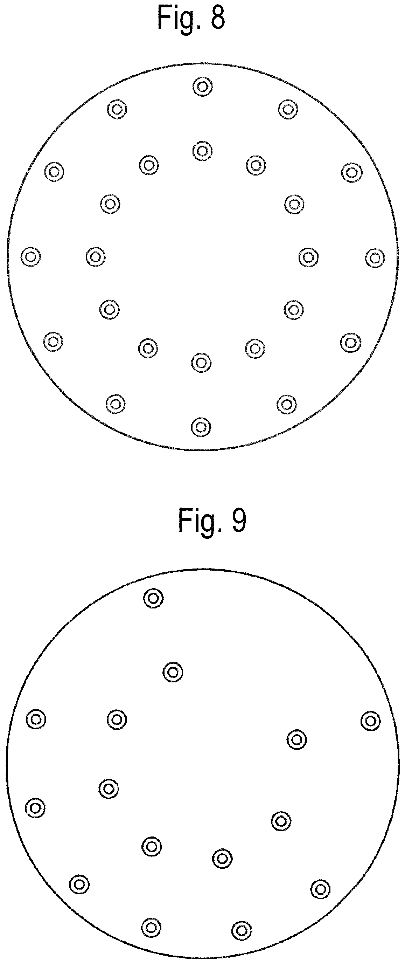

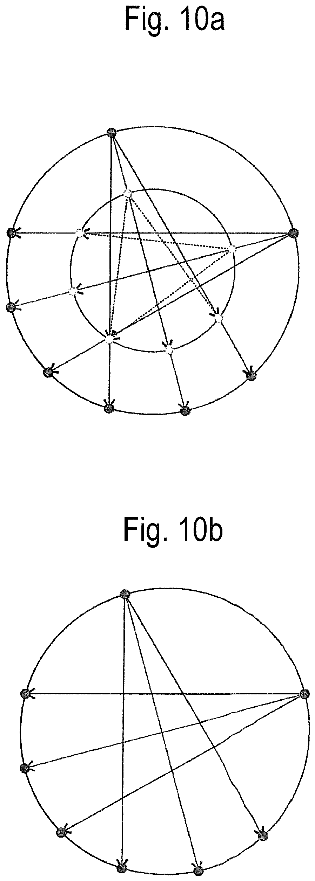

Die in Fig. 8, 9 sowie 10a und 10b dargestellte Varianten einer Sensoranordnung mit den dargestellten Ausrichtungen der Elektrodenachsen erlauben eine Virtuelle Drehung der Sensoren, indem die Elektrodenzuordnung einer orthogonalen Achsenausrichtung geändert wird. Hierfür werden zusätzlich zu den beiden senkrecht zueinander ausgerichteten Elektrodenanordnungen zumindest eine weitere, vorzugsweise jedoch zwei Elektrodenachsen, durch Positionierung weiterer Elektroden vorgegeben. Wobei die Elektrodenachsen, ausgehend von einer Elektrode als Winkelscheitel beider Achsen, in einem Winkel, vorzugsweise von zumindest 5 Grad, besonders bevorzugt von zumindest 10 Grad, zueinander angeordnet sind. Auf den vorgenannten Achsen befinden sich somit zusätzlich zur Elektrode im Winkelscheitel jeweils zumindest zwei der Elektroden der jeweiligen Elektrodenanordnung. Die Achsen sind insbesondere aus Fig. 10a und 10b gut erkennbar.

Die in den vorangegangenen Figuren dargestellten Elektroden haben vorrangig einen runden Querschnitt. Es ist allerdings im Rahmen der vorliegenden Erfindung auch möglich, dass andere Elektrodenquerschnitte, z.B. rechteckige Elektrodenquerschnitte und/oder Kontaktflächen, realisiert werden.

### Bezugszeichen

- 1: Messgerät
- 2: Armband
- 3: Display
- 3.1 - 3.5: Bedienelement
- 4: Messumformer
- 5: Sensoreinheit
- 6: Elektrodenanordnung
- 6.1: Elektrode
- 6.2: Elektrode
- 6.3: Elektrode
- 6.4: Elektrode
- 7: Elektrodenanordnung
- 8: Elektrodenanordnung
- 9: Elektrodenanordnung
- 10: Mittelpunkt
- 11: Platte
- 12: Kontaktfläche
- 13: Leitung
- 14: erste Kreisbahn
- 15: zweite Kreisbahn
- 20: Elektrode
- 21: Innenelektrode
- 22: Elektrodenschaft
- 23: Vorsprung
- 24: Kontaktfläche
- 25: Signalleitung
- 26: Lötverbindung
- 27: Stützhülse
- 28: Kanal
- 29: Auskragung
- 31: Grundplatte
- 32: Rand
- 33: Pfeil
- 34: Elektronikplatine
- 35: Kontaktflächen
- 36: Kreisbahn
- 37: Kreisbahn
- 41: Grundplatte
- 44: Leiterplatte
- 46: Trägerplatte
- 51: Grundplatte
- 52: Ringplatte
- 53: Leiterplatte
- 54: Kontaktfläche
- 56: innere Kreisbahn
- 57: äußere Kreisbahn

## Patentansprüche

1. Messgerät (1) zur Ermittlung eines Schätzwertes für den Blutzuckergehalt einer Person durch eine noninvasive Impedanzmessung, wobei das Messgerät (1) einen Messumformer (4) zur Ermittlung eines Blutzuckergehalts aus einem Messwert aufweist und eine Sensoreinheit (5) zum Erfassen des Messwerts aufweist,
wobei die Sensoreinheit (5) zumindest zwei tetrapolare Elektrodenanordnungen (6-9) aufweist mit jeweils zumindest zwei Elektrodenpaaren (6.1, 6.2 und 6.3, 6.4), welche entlang einer ersten linearen Achse angeordnet sind, wobei die Achsen der zwei tetrapolaren Elektrodenanordnungen (6, 8 oder 7, 9) senkrecht zueinander ausgerichtet sind und wobei ein erstes Elektrodenpaar (6.1, 6.2) zum Aussenden und Empfang eines Stromsignals vorgesehen ist und wobei ein zweites Elektrodenpaar (6.3, 6.4) zum Abgriff eines Potentials bei Kontakt mit der Haut der Person vorgesehen ist,
**dadurch gekennzeichnet, dass**
zumindest eine der Elektroden (6.1-6.4, 20) der Sensoreinheit (5) eine Kontaktfläche (12, 24) zur Kontaktierung mit der Haut aufweisen, welche aus einem Metall oder einer Metalllegierung mit einer Leitfähigkeit von mehr als 1*10⁷ S/m besteht, und die Elektrode (6.1-6.4, 20) eine Innenelektrode (21) aufweist, welche aus einem Edelmetall besteht, und eine Stützhülse (27) aufweist aus einem Material mit einer Vickershärte von mehr als 40, gemäß der DIN EN 6507-1:2018-07, oder aus einer Siliziumverbindung mit einer Vickershärte von mehr als 40, gemäß der DIN EN 6507-1:2018-07 und mit einer Wärmeleitfähigkeit von mehr als 100 W/(m*K).

2. Messgerät nach Anspruch 1, **dadurch gekennzeichnet, dass** das Metall Gold, Rhodium, Tantal, Iridium und/oder Osmium ist oder dass die Metalllegierung zumindest zu 40 Gew.% aus einem oder mehreren der vorgenannten Metalle gebildet ist.

3. Messgerät nach Anspruch 1 oder 2, **dadurch gekennzeichnet, dass** die Innenelektrode (21) aus Gold und die Stützhülse (27) aus Titan, Stahl, alpha Siliziumcarbid oder aus einer berylliumhaltigen Keramik besteht.

4. Messgerät nach einem der vorhergehenden Ansprüche, **dadurch gekennzeichnet, dass** die Elektrode (6.1-6.4) einen Elektrodenkörper aus Kupfer und/oder Messing und/oder alpha Siliziumcarbid aufweist, wobei der Elektrodenkörper mit einer Beschichtung aus Rhodium, Tantal, Iridium und/oder Osmium unter Ausbildung der Kontaktfläche versehen ist.

5. Messgerät nach Anspruch 4, **dadurch gekennzeichnet, dass** die Beschichtungsdicke zumindest 2 µm, vorzugsweise zwischen 3 bis 10 µm, beträgt.

6. Messgerät nach einem der vorhergehenden Ansprüche, **dadurch gekennzeichnet, dass** die Sensoreinheit (5) eine Platte (11) oder ein Ledersegment aufweist, auf welcher oder welchem die tetrapolaren Elektrodenanordnungen (6-9) festgelegt, insbesondere verankert, sind wobei die Platte (11) oder das Ledersegment eine Dauerbiegefestigkeit nach DIN EN ISO 5402-1:2017-05 oder ISO 4666-2 von 100.000 Knickungen ohne Beschädigung aufweist.

7. Messgerät nach einem der vorhergehenden Ansprüche, **dadurch gekennzeichnet, dass** jeweils eine Elektrode (6.1-6.4, 20) in einer Öffnung der Platte (11) und/oder des Lederstreifens angeordnet ist, wobei die Stützhülse (27) eine radiale Auskragung (29) als Anschlag auf der Oberfläche der Platte (11) oder des Lederstreifens randseitig zu der Öffnung aufweist.

8. Messgerät nach einem der vorhergehenden Ansprüche, **dadurch gekennzeichnet, dass** die Breite, insbesondere der Durchmesser, der Kontaktfläche (12, 24) der Elektrode (6.1-6.4, 20), insbesondere der Innenelektrode (21), geringer ist als 5 mm, vorzugsweise 2-3 mm.

9. Messgerät nach einem der vorhergehenden Ansprüche, **dadurch gekennzeichnet, dass** die Elektrode (6.1-6.4, 20) oder zumindest die Innenelektrode (21) als Rundelektrode, insbesondere als Pilzkopfelektrode, ausgebildet ist.

10. Messgerät nach einem der vorhergehenden Ansprüche, **dadurch gekennzeichnet, dass** die Sensoreinheit (5) vier oder acht tetrapolare Elektrodenanordnungen (6-9) mit jeweils zwei Elektrodenpaaren (6.1, 6.2 und 6.3, 6.4), welche auf einer linearen Achse angeordnet sind, aufweist.

11. Messgerät nach einem der vorhergehenden Ansprüche, **dadurch gekennzeichnet, dass** die Elektroden (6.1-6.4, 20) der Sensoreinheit (5) auf zwei, vorzugsweise konzentrisch-zueinander angeordneten, Kreisbahnen (14, 15) angeordnet ist, wobei die Zahl der Elektroden (6.1, 6.2, 20) auf der ersten Kreisbahn (14) gleich der Zahl der Elektroden (6.3, 6.4, 20) auf der zweiten Kreisbahn (15) ist.

12. Messgerät nach einem der vorhergehenden Ansprüche, **dadurch gekennzeichnet, dass** der Durchmesser der ersten Kreisbahn (14) zwischen 20 bis 40 mm beträgt und wobei der Durchmesser der zweiten Kreisbahn (15) zumindest um 30% kleiner ist als der Durchmesser der ersten Kreisbahn (14).

13. Messgerät nach einem der vorhergehenden Ansprüche, **dadurch gekennzeichnet, dass** der Abstand zweier benachbarter Elektroden (6.1 oder 6.2) zweier benachbarter Elektrodenanordnungen auf der ersten Kreisbahn (14) mehr als 4 mm, vorzugsweise zwischen 5 und 8 mm, beträgt.

14. Messgerät nach einem der vorhergehenden Ansprüche, **dadurch gekennzeichnet, dass** die Anordnung (6) der Elektroden der Sensoreinheit (5) punktsymmetrisch und insbesondere spiegelsymmetrisch ist.

15. Messgerät nach einem der vorhergehenden Ansprüche, **dadurch gekennzeichnet, dass** das Messgerät (1) ein portables Messgerät ist, ein Armband (2) und/oder Fußband aufweist, wobei die Sensoreinheit (5) eine Breite parallel zur Armband- und/oder Fußbandbreite von weniger als 40 mm, vorzugsweise zwischen 15-35 mm, aufweist.

16. Messgerät nach einem der vorhergehenden Ansprüche, **dadurch gekennzeichnet, dass** die Sensoreinheit eine starr mit der Person verbindbares, vorzugsweise plattenförmiges, Element (34, 44, 53) aufweist, wobei die Achsen der Elektrodenanordnungen (6, 8) gegenüber dem plattenförmigen Element (34, 44, 53) drehbar, vorzugsweise um eine Flächennormale der Oberfläche des Elements (34, 44, 53), angeordnet sind.

17. Messgerät nach einem der vorhergehenden Ansprüche, **dadurch gekennzeichnet, dass** das Element (34, 44, 53) kreisbogenförmige Kontaktflächen (35, 54) zum Signalabgriff der Elektroden der Elektrodenanordnungen (6, 8) aufweist.

18. Messgerät nach einem der vorhergehenden Ansprüche, **dadurch gekennzeichnet, dass** die Achse einer der tetrapolaren Elektrodenanordnungen mit einer zweiten Achse einer weiteren Elektrodenanordnung, ausgehend von einer gemeinsamen Elektrode als Winkelscheitel winklig zueinander angeordnet sind, wobei der Signalabgriff durch eine Steuereinheit derart gesteuert wird, dass zum Zweck einer virtuellen Drehung der Elektrodenanordnung wahlweise die Signale an den Elektroden der ersten oder der zweiten Achse abgegriffen werden.

## Claims

1. Measuring device (1) for determining an estimated value for the blood sugar content of a person by means of a non-invasive impedance measurement, wherein the measuring device (1) has a transmitter (4) for determining a blood sugar content from a measured value and having a sensor unit (5) for detecting the measured value,
wherein the sensor unit (5) has at least two tetrapolar electrode arrangements (6-9), each having at least two electrode pairs (6.1, 6.2 and 6.3, 6.4) which are arranged along a first linear axis, wherein the axes of the two tetrapolar electrode arrangements (6, 8 or 7, 9) are aligned perpendicular to one another and wherein a first pair of electrodes (6.1, 6.2) is provided for emitting and receiving a current signal and wherein a second pair of electrodes (6.3, 6.4) is provided for tapping a potential on contact with the skin of the person,
**characterized in that**
at least one of the electrodes (6.1-6.4, 20) of the sensor unit (5) has a contact surface (12, 24) for contacting the skin, which consists of a metal or a metal alloy with a conductivity of more than 1*10⁷ S/m, and the electrode (6.1-6.4, 20) has an inner electrode (21) which consists of a precious metal and has a support sleeve (27) made of a material with a Vickers hardness of more than 40, according to DIN EN 6507-1:2018-07, or of a silicon compound with a Vickers hardness of more than 40, according to DIN EN 6507-1:2018-07, and with a thermal conductivity of more than 100 W/(m*K).

2. Measuring device according to claim 1, **characterized in that** the metal is gold, rhodium, tantalum, indium and/or osmium, or **in that** the metal alloy consists of at least 40 wt.% from one or more of the aforementioned metals.

3. Measuring device according to claim 1 or 2, **characterized in that** the inner electrode (21) consists of gold and the support sleeve (27) consists of titanium, steel, alpha silicon carbide or of a beryllium-containing ceramic.

4. Measuring device according to one of the preceding claims, **characterized in that** the electrode (6.1-6.4) has an electrode body made of copper and/or brass and/or alpha silicon carbide, wherein the electrode body is provided with a coating of rhodium, tantalum, indium and/or osmium to form the contact surface.

5. Measuring device according to claim 4, **characterized in that** the coating thickness is at least 2 µm, preferably between 3 and 10 µm.

6. Measuring device according to one of the preceding claims, **characterized in that** the sensor unit (5) has a plate (11) or a leather segment on which the tetrapolar electrode arrangements (6-9) are fixed, in particular anchored, wherein the plate (11) or the leather segment has a fatigue bending strength according to DIN EN ISO 5402-1:2017-05 or ISO 4666-2 of 100,000 folds without damage.

7. Measuring device according to one of the preceding claims, **characterized in that** in each case one electrode (6.1-6.4, 20) is arranged in an opening of the plate (11) and/or of the leather strip, wherein the supporting sleeve (27) has a radial projection (29) as a stop on the surface of the plate (11) or of the leather strip at the edge of the opening.

8. Measuring device according to one of the preceding claims, **characterized in that** the width, in particular the diameter, of the contact surface (12, 24) of the electrode (6.1-6.4, 20), in particular of the inner electrode (21), is less than 5 mm, preferably 2-3 mm.

9. Measuring device according to one of the preceding claims, **characterized in that** the electrode (6.1-6.4, 20) or at least the inner electrode (21) is designed as a round electrode, in particular as a mushroom head electrode.

10. Measuring device according to one of the preceding claims, **characterized in that** the sensor unit (5) has four or eight tetrapolar electrode arrangements (6-9) each with two pairs of electrodes (6.1, 6.2 and 6.3, 6.4), which are arranged on a linear axis.

11. Measuring device according to one of the preceding claims, **characterized in that** the electrodes (6.1-6.4, 20) of the sensor unit (5) are arranged on two circular paths (14, 15), which are preferably arranged concentrically to one another, wherein the number of electrodes (6.1, 6.2, 20) on the first circular path (14) is equal to the number of electrodes (6.3, 6.4, 20) on the second circular path (15).

12. Measuring device according to one of the preceding claims, **characterized in that** the diameter of the first circular path (14) is between 20 and 40 mm and wherein the diameter of the second circular path (15) is at least 30% smaller than the diameter of the first circular path (14).

13. Measuring device according to one of the preceding claims, **characterized in that** the distance between two adjacent electrodes (6.1 or 6.2) of two adjacent electrode arrangements on the first circular path (14) is more than 4 mm, preferably between 5 and 8 mm.

14. Measuring device according to one of the preceding claims, **characterized in that** the arrangement (6) of the electrodes of the sensor unit (5) is point-symmetrical and in particular mirror-symmetrical.

15. Measuring device according to one of the preceding claims, **characterized in that** the measuring device (1) is a portable measuring device, has a wristband (2) and/or ankle band, wherein the sensor unit (5) has a width parallel to the wristband and/or ankle band width of less than 40 mm, preferably between 15-35 mm.

16. Measuring device according to one of the preceding claims, **characterized in that** the sensor unit has a preferably plate-shaped element (34, 44, 53) which can be rigidly connected to the person, wherein the axes of the electrode arrangements (6, 8) are arranged rotatably with respect to the plate-shaped element (34, 44, 53), preferably about a surface normal of the surface of the element (34, 44, 53).

17. Measuring device according to one of the preceding claims, **characterized in that** the element (34, 44, 53) has circular arc-shaped contact surfaces (35, 54) for signal tapping of the electrodes of the electrode arrangements (6, 8).

18. Measuring device according to one of the preceding claims, **characterized in that** the axis of one of the tetrapolar electrode arrangements is arranged at an angle to one another with a second axis of a further electrode arrangement, starting from a common electrode as an angular vertex, wherein the signal tapping is controlled by a control unit in such a way that, for the purpose of a virtual rotation of the electrode arrangement, the signals are selectively tapped at the electrodes of the first or the second axis.

## Revendications

1. Appareil de mesure (1) destiné à déterminer une valeur d'estimation pour la glycémie d'une personne par mesure de l'impédance de façon non invasive, l'appareil de mesure (1) présentant un transducteur de mesure (4) destiné à déterminer une glycémie à partir d'une valeur mesurée, et présentant une unité de capteur (5) destinée à enregistrer la valeur mesurée
l'unité de capteur (5) présentant au moins deux agencements d'électrodes tétrapolaires (6-9) comportant respectivement au moins deux paires d'électrodes (6.1, 6.2 et 6.3, 6.4), qui sont agencées le long d'un premier axe linéaire, les axes des deux agencements d'électrodes tétrapolaires (6, 8 ou 7, 9) étant perpendiculaires l'un à l'autre, et une première paire d'électrodes (6.1, 6.2) étant prévu à émettre et à recevoir un signal de courant, et une seconde paire d'électrodes (6.3, 6.4) étant prévu à prélever un potentiel lors du contact avec la peau de la personne,
**caractérisé en ce que**
au moins l'une des électrodes (6.1-6.4, 20) de l'unité de capteur (5) présente une surface de contact (12, 24) destinée à venir en contact avec la peau, qui est constituée d'un métal ou d'un alliage métallique de conductivité supérieure à 1*10⁷ S/m, et l'électrode (6.1-6.4, 20) présente une électrode interne (21) qui est constituée d'un métal précieux, et présente une douille de support (27) constituée d'un matériau ayant une dureté Vickers supérieure à 40, selon la norme DIN EN 6507-1:2018-07, ou d'un composé de silicium ayant une dureté Vickers supérieure à 40, selon la norme DIN EN 6507-1:2018-07 et ayant une conductivité thermique supérieure à 100 W/(m*K).

2. Appareil de mesure selon la revendication 1, **caractérisé en ce que** le métal est l'or, le rhodium, le tantale, l'iridium et/ou l'osmium ou **en ce que** l'alliage métallique est formé au moins à 40% en poids d'un ou de plusieurs des métaux susmentionnés.

3. Appareil de mesure selon la revendication 1 ou 2, **caractérisé en ce que** l'électrode interne (21) est en or et la douille de support (27) est en titane, en acier, en carbure de silicium alpha ou en une céramique contenant de l'humus de béryl.

4. Appareil de mesure selon l'une des revendications précédentes, **caractérisé en ce que** l'électrode (6.1-6.4) présente un corps d'électrode en cuivre et/ou en laiton et/ou en carbure de silicium alpha, le corps d'électrode étant pourvu d'un revêtement en rhodium, en tantale, en iridium et/ou en osmium en formant la surface de contact.

5. Appareil de mesure selon la revendication 4, **caractérisé en ce que** l'épaisseur du revêtement est d'au moins 2 µm, de préférence comprise entre 3 et 10 µm.

6. Appareil de mesure selon l'une des revendications précédentes, **caractérisé en ce que** l'unité de capteur (5) présente une plaque (11) ou un segment de cuir sur lequel ou laquelle les agencements d'électrodes tétrapolaires (6-9) sont fixés, en particulier ancrés, la plaque (11) ou le segment de cuir présentant une résistance à la flexion permanente selon DIN EN ISO 5402-1:2017-05 ou ISO 4666-2 de 100 000 pliages sans détérioration.

7. Appareil de mesure selon l'une des revendications précédentes, **caractérisé en ce qu'**une électrode (6.1-6.4, 20) est disposée dans une ouverture de la plaque (11) et/ou de la bande de cuir, la douille de support (27) présentant une saillie radiale (29) servant de butée sur la surface de la plaque (11) ou de la bande de cuir du côté du bord de l'ouverture.

8. Appareil de mesure selon l'une des revendications précédentes, **caractérisé en ce que** la largeur, notamment le diamètre, de la surface de contact (12, 24) de l'électrode (6.1-6.4, 20), notamment de l'électrode interne (21), est inférieure à 5 mm, de préférence à 2-3 mm.

9. Appareil de mesure selon l'une des revendications précédentes, **caractérisé en ce que** l'électrode (6.1-6.4, 20) ou au moins l'électrode interne (21) est conçue comme une électrode ronde, en particulier comme une électrode en forme de tête de champignon.

10. Appareil de mesure selon l'une des revendications précédentes, **caractérisé en ce que** l'unité de capteur (5) présente quatre ou huit agencements d'électrodes tétrapolaires (6-9) présentant chacun deux paires d'électrodes (6.1, 6.2 et 6.3, 6.4) disposées sur un axe linéaire.

11. Appareil de mesure selon l'une des revendications précédentes, **caractérisé en ce que** les électrodes (6.1-6.4, 20) de l'unité de capteur (5) sont disposées sur deux trajectoires circulaires (14, 15), de préférence disposées concentriques l'une à l'autre, le nombre d'électrodes (6.1, 6.2, 20) sur la première trajectoire circulaire (14) étant égal au nombre d'électrodes (6.3, 6.4, 20) sur la deuxième trajectoire circulaire (15).

12. Appareil de mesure selon l'une des revendications précédentes, **caractérisé en ce que** le diamètre de la première trajectoire circulaire (14) est compris entre 20 et 40 mm, et dans lequel le diamètre de la deuxième trajectoire circulaire (15) est inférieur d'au moins 30% au diamètre de la première trajectoire circulaire (14).

13. Appareil de mesure selon l'une des revendications précédentes, **caractérisé en ce que** la distance entre deux électrodes voisines (6.1 ou 6.2) de deux agencements d'électrodes voisins sur la première trajectoire circulaire (14) est supérieure à 4 mm, de préférence comprise entre 5 et 8 mm.

14. Appareil de mesure selon l'une des revendications précédentes, **caractérisé en ce que** l'agencement (6) des électrodes de l'unité de capteur (5) présente une symétrie ponctuelle et notamment une symétrie spéculaire.

15. Appareil de mesure selon l'une des revendications précédentes, **caractérisé en ce que** l'appareil de mesure (1) est un appareil de mesure portable, présente un bracelet (2) et/ou une chevillère, l'unité de capteur (5) ayant une largeur parallèle à la largeur du bracelet et/ou de la chevillère inférieure à 40 mm, de préférence comprise entre 15 et 35 mm.

16. Appareil de mesure selon l'une des revendications précédentes, **caractérisé en ce que** l'unité de capteur présente un élément (34, 44, 53), de préférence en forme de plaque, pouvant être relié de manière rigide à la personne, les axes des agencements d'électrodes (6, 8) étant disposés de manière rotative par rapport à l'élément (34, 44, 53) en forme de plaque, de préférence autour d'une normale de surface à la surface de l'élément (34, 44, 53).

17. Appareil de mesure selon l'une des revendications précédentes, **caractérisé en ce que** l'élément (34, 44, 53) présente des surfaces de contact en arc de cercle (35, 54) pour prélever le signal des électrodes des agencements d'électrodes (6, 8).

18. Appareil de mesure selon l'une des revendications précédentes, **caractérisé en ce que** l'axe de l'un des ensembles d'électrodes tétrapolaires est disposé angulairement l'un par rapport à l'autre avec un deuxième axe d'un autre ensemble d'électrodes, en partant d'une électrode commune comme sommet d'angle, le prélèvement de signal étant commandé par une unité de commande de telle sorte que, dans le but d'une rotation virtuelle de l'ensemble d'électrodes, les signaux sont prélevés au choix sur les électrodes du premier ou du deuxième axe.
